(19) Europäisches Patentamt
European Patent Office
Office européen des brevets

(11) EP 2 259 045 A1

(12) EUROPEAN PATENT APPLICATION

(43) Date of publication:
08.12.2010 Bulletin 2010/49

(51) Int Cl.:
G01N 15/10 (2006.01) G01N 33/50 (2006.01)

(21) Application number: 09162096.3

(22) Date of filing: 05.06.2009

(84) Designated Contracting States:
AT BE BG CH CY CZ DE DK EE ES FI FR GB GR HR HU IE IS IT LI LT LU LV MC MK MT NL NO PL PT RO SE SI SK TR
Designated Extension States:
AL BA RS

(71) Applicant: Koninklijke Philips Electronics N.V.
5621 BA Eindhoven (NL)

(72) Inventor: **The designation of the inventor has not yet been filed**

(74) Representative: **Damen, Daniel Martijn**
**Philips**
**Intellectual Property & Standards**
**P.O. Box 220**
**5600 AE Eindhoven (NL)**

(54) **Multi-frequency impedance method and apparatus for discriminating and counting particles expressing a specific marker**

(57)     An analysis method for identifying and counting particles expressing a specific antigenic marker is proposed. One example (but not exclusive) is CD4+ T-lymphocyte analysis in blood. The method comprises obtaining a sample comprising particles in suspension, labeling the particles expressing the specific marker with an impedance label, and measuring the labeled sample using a dual frequency system to discriminate at least the labeled particles expressing the specific marker. The method allows an accurate count of particles e.g. the number CD4+ T-lymphocytes in a blood flow. A corresponding device is also provided.

FIG. 1

Printed by Jouve, 75001 PARIS (FR)

**Description**

FIELD OF THE INVENTION

The present invention relates to the field of medical devices, medical diagnostics, and particle counting, and more specifically to a method and device for discriminating, identifying and counting different particles, including bacteria, viruses, non-biological particles and cells. One example could be e.g. blood cells, expressing a specific marker, e.g. for discriminating, identifying and counting CD4+ T-lymphocytes in human blood. Another example could be bacteria or viruses expressing different surface markers. Another example might be a solid particle with a surface marker, which could be an antibody, or piece of DNA or peptide.

BACKGROUND OF THE INVENTION

Microfluidic systems have shown unique promise for studying cell function, cell and tissue engineering, disease diagnosis, blood sample preparation, and drug discovery. Very recently the use of microfluidics to isolate pure populations of leukocyte or white blood cell subsets from whole blood has attracted a lot of interest for point-of-care diagnosis.

As an example, the diagnosis of immunodeficiency disease, primarily HIV, is performed using blood analysis. Determination of the concentration of T-helper lymphocytes in blood is important for monitoring the progression of an HIV infection. Therefore it is important to detect the presence of and count CD4+ and CD8+ T-lymphocytes. According to WHO guidelines, a CD4+ T-cell count of less than 200 cells/$\mu$l of blood establishes a positive diagnosis of AIDS and in most settings is used as an indication for the need to apply antiretroviral treatment. Initiation of antiretroviral treatment before the CD4+ count falls below 200 cells/$\mu$l, e.g. as from a threshold of 350 cells/$\mu$l, is recommended, and a CD4+ count threshold of 500 cells/$\mu$l is widely used to increase the frequency of clinical monitoring of patients.

The quantification of CD4+ T-lymphocyte levels by fluorescence based flow cytometry (FACS) is currently the gold standard. Fluorescence based flow cytometers are complex and expensive pieces of equipment and, as such, have not penetrated into resource-poor settings such as sub-Saharan Africa. There is a need for a simple to use point-of-care CD4+ T-cell counting system, for example for use in the developing world.

Impedance spectroscopy (IS) has been widely used to measure the dielectric properties of cells, providing values for membrane capacitance, membrane resistance, cytoplasmic conductivity and permittivity. It can also be considered a "label-free" method of analysis. Generally cells are measured in suspension, and the data represent the average for the population. The first high-speed measurements of the electrical properties of single cells were performed by Coulter in the 1950s. It was shown that individual blood cells could be counted and discriminated using an "electrical volume measurement" performed with a single low-frequency (or DC) electrical signal. In the 1970s Hoffman and Britt developed a macro-system for simultaneous low and high frequency impedance analysis of single cells using a miniature flow cell that had electrodes placed in a flow channel.

X. Cheng et al., in "Cell detection and counting through cell lysate impedance spectroscopy in microfluidic devices", Lab Chip, 2007, 7, 746-755, describe an electrical method for counting cells based on the measurement of changes in conductivity of the surrounding medium due to ions released from surface-immobilized cells within a microfluidic channel. Immobilized cells are lysed using a low conductivity, hypotonic media and the resulting change in impedance is measured using surface patterned electrodes to detect and quantify the number of cells. The method of cell lysate as described is sensitive enough to detect 20 cells $\mu$l$^{-1}$, and offers a simple and efficient method for detecting and enumerating cells within microfluidic devices for many applications, including measurement of CD4 cell counts in HIV patients in resource-limited settings.

Impedance spectroscopy is generally performed on a large population of cells, usually of mixed type. Analysis of CD4+ cells in a suspension of other cells is therefore not possible using this method. CD4+ cells can be immobilized on a substrate and counted by microscope, but this is not very reliable.

While the principle behind a cell isolation approach can be easily adapted to a wide spectrum of clinical applications, detecting these isolated cells remains a technical challenge to be addressed.

SUMMARY OF THE INVENTION

It is an object of embodiments of the present invention to provide a good method and device for identifying and counting particles, such as bacteria, viruses, non-biological particles or cells, e.g. blood cells, expressing a specific marker. A good method and device according to embodiments of the present invention is accurate in that the number of identified particles expressing a specific marker can be accurately determined. Furthermore, a good method and device according to embodiments of the present invention could also be used in resource-poor settings, without, however, being limited thereto. Other areas of application include PoC devices for generic analysis and identification of different cells expressing different markers.

The above objective is accomplished by a method and device according to the present invention.

The present invention provides a general method and device for discriminating, identifying and counting particles, in particular embodiments blood cells, expressing a specific marker, for example a specific antigenic marker. This is obtained by using impedance labeling in combination with plural frequency (two or more frequencies) impedance cytometry, optionally for diagnosis of disease.

In a first aspect, the present invention provides an analysis method for identifying and counting particles, e.g. bacteria, viruses, non-biological particles or cells, such as for example blood cells, expressing a specific marker or other antibody. As an example only, the method may be applied to identify and count CD4+ T-lymphocytes in blood, The method comprises:

obtaining a sample of particles in suspension, for example blood comprising blood cells,
labeling the particles, e.g. blood cells, expressing the specific marker with an impedance label, and
measuring the labeled sample using a plural frequency system to discriminate at least the labeled particles expressing the specific marker.

It is an advantage of a method according to embodiments of the present invention that it allows a fast, inexpensive and simple analysis, e.g. full blood cell analysis (full blood count) can be performed in a few minutes. A method according to embodiments of the present invention can be used routinely in clinical practice. Only a limited amount of sample, e.g. blood, is required, as the analysis is performed on individual particles (or individual particle/label complexes) rather than on a large population of particles. To achieve a pre-determined number of counting events, a given amount of sample, e.g. blood, must be used. A method according to embodiments of the present invention does not change the number of particles in a drop of sample, e.g. the number of cells in a drop of blood. However, state of the art systems use venipuncture blood in 4ml tubes because they are used in large central laboratories and the 4ml tubes is how samples are sent to the lab. In accordance with embodiments of the present invention, the method may be applied in a microfluidic device, and the microfluidic format allows integrated sample preparation which in turn enables point of care use, hence allowing immediate access to the sample requiring no storage thereof. The fact that a finger prick of blood contains enough cells to make the count statistically significant, permits the use of only a finger prick of blood rather than a 4 ml tube.

In an analysis method according to embodiments of the present invention, labeling the particles expressing the specific marker may be performed within a microfluidic system. In particular embodiments, the method may be applied to e.g. blood cells within a microfluidic system. The cells only need to be added into the microfluidic system, and a lysis and the labeling could be done sequentially.

In an analysis method according to embodiments of the present invention, the step of measuring the labeled sample to discriminate at least the labeled particles expressing the specific marker may comprise splitting the labeled sample into droplets comprising a single particle, determining the impedance of the single particle at least at a first frequency and at a second frequency, optionally at more than two frequencies, the first frequency being lower than the second frequency, and correlating the impedance at the first, lower, frequency to the opacity, the opacity being defined at the ratio of the second, higher, frequency impedance to the first, lower, frequency impedance, and from this correlation discriminating at least the labeled particles expressing the specific marker.

In an analysis method according to embodiments of the present invention, labeling a sample may include incubation of the sample with beads coated with an antibody to the specific marker.

A method according to embodiments of the present invention may furthermore comprise, before measuring the labeled sample, performing a lysis step. Such lysis step allows an easier discrimination of cells expressing a specific marker. It is particularly advantageous that the lysis step may be performed in a microfluidic device. In case the sample is blood, the lysis step may comprise an erythrocyte lysis step. Such erythrocyte lysis step may comprise exposing the blood sample to a saponin in an organic acid.

In embodiments of the present invention, measuring the labeled sample may be performed in a sample flow, for example in a microfluidic device.

In a second aspect, the present invention provides the use of a method according to embodiments of the present invention for identifying and counting particles in a sample. In a particular embodiment of the second aspect, the present invention provides the use of a method according to embodiments of the present invention for detection and counting of CD4+ T-lymphocytes. Such count is advantageous as it helps in diagnosing HIV. In embodiments of the present invention the number of individual CD4+ cells is estimated from the impedance signal of single cells.

In a third aspect, the present invention provides a device for identifying and counting particles expressing a specific marker, comprising a micro-channel for comprising a sample flow, e.g. particles in suspension, comprising labeled particles expressing the specific marker, at least one pair of electrodes, electronic circuitry, such as for example a function generator, for applying at least a first AC signal at a first frequency and a second AC signal at a second frequency to the at least one pair of electrodes, the first frequency being lower than the second frequency, and a calculation unit for determining the impedance of a particle present between the pair of electrodes, the calculation unit being adapted for determining the impedance of the particle at least at the first, lower, frequency and at the second, higher, frequency, for correlating the impedance at the first, lower, frequency to the opacity being defined as the ratio of the second, higher, frequency impedance to the first, lower, frequency impedance, and for discriminating from this correlation at least the labeled particles expressing the specific marker.

Such device according to embodiments of the present invention is different from the prior art, where particles, e.g. blood cells, expressing the specific marker are immobilized on a fixed substrate, hence the micro-channel does not comprise a flow of sample.

A device according to embodiments of the present invention may furthermore comprise a second pair of electrodes adapted for functioning as reference electrodes.

A device according to embodiments of the present invention may furthermore comprise an amplifier circuit adapted for making a differential measurement on a particle present between one of the pairs of electrodes. This way, a microfluidic single-cell impedance cytometer is provided that is able to perform a differential count, for example in case the sample is a blood sample, a white blood cell differential count.

In a device according to embodiments of the present invention, the micro-channel may be adapted for receiving a flow of particles in suspension and for directing the flow of particles in suspension between the at least one pair of electrodes for identifying and counting in the flow of particles in suspension those particles expressing a specific marker.

In a device according to embodiments of the present invention, the low frequency measurement capabilities can be extended so as to allow detection of biological particles, such as for example DNA, virus, proteins etc., present in a sample through modification of the impedance properties of the functionalized bead used for labeling the particles.

In accordance with embodiments of the present invention, a microfluidic device could be scaled down to allow for the detection of binding of biological particles, e.g. DNA, virus, proteins etc. to functionalized nano-sccale beads.

It is an advantage of embodiments of the present invention that they may be used for CD4+ T-lymphocyte counting. It is an advantage of embodiments of the present invention that they are simple to use, fast, accurate and inexpensive.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other aspects of the invention will be apparent from and elucidated with reference to the embodiment(s) described hereinafter.

BRIEF DESCRIPTION OF THE DRAWINGS

Fig. 1 is a schematic diagram of a micro impedance cytometer system according to embodiments of the present invention, implementing dual frequency impedance measurement.

Fig. 2(a) is a simplified schematic of the impedance detection system according to embodiments of the present invention, showing the differential detection system. The electrode pair on the left illustrates the equivalent circuit model (ECM) without a cell between the electrodes, the electrode pair on the right has the cell present between the electrodes.Fig. 2(b) illustrates a comparison of a typical frequency-dependent impedance magnitude signal for a polymer bead and for a cell of similar size.

Fig. 3 gives experimental data showing the magnitude of the impedance versus frequency for (a) 5.62 $\mu$m diameter latex beads, and (b) MACS purified populations of T-lymphocytes, monocytes, neutrophils. Each point comprises about 1500 events and shows the mean and standard deviation of the measurement data. The dashed lines show the PSpice circuit simulation incorporating the equivalent circuit model for the detection region with a cell (or bead) and the differential amplification electronics.

Fig. 4(a) is a scatter plot showing the opacity ($|Z|$ @ 1707 kHz / $|Z|$ @ 503 kHz) plotted against the low frequency (503 kHz) impedance magnitude for a mixture of T-lymphocytes, monocytes, neutrophils and a population of 5.62 $\mu$m diameter latex beads.

Fig. 4(b) shows a histogram of the low frequency impedance magnitude.

Fig. 4(c) shows a histogram of the opacity.

Fig. 4(d) illustrates a FACS analysis of the same sample showing the forward and side scatter properties of the cells (without beads).

Fig. 5(a)-(c) are FACS scatter plots showing the fluorescence and forward light scatter properties of the fluorescent labeled (anti-CD14-FITC and anti-CD 16-Alexa700) saponin/formic acid treated whole blood. All the events were triggered on the FSC signal, cell populations identified according to their fluorescence signal.

Fig. 5(d)-(f) are scatter plots (from the same sample) showing the fluorescence and low frequency (503 kHz) impedance magnitude properties as measured using the micro impedance cytometer. The data show that both systems can clearly resolve the three cell populations neutrophils, monocytes and lymphocytes.

Fig. 6(a) is a scatter plot showing the opacity (|Z| @ 1707 kHz / |Z| @ 503 kHz) versus the low frequency (503 kHz) impedance magnitude for saponin/formic acid treated whole blood and a population of 7.18 μm diameter latex beads.

Fig. 6(b) shows a histogram of the low frequency impedance magnitude. Data for the separate populations are fitted to Gaussian distributions (grey curves).

Fig. 6(c) shows a histogram of the opacity. Data for the separate populations are fitted to Gaussian distributions (grey curves).

Fig. 6(d) is a FACS analysis of the same sample showing the forward and side scatter properties of the cells (without beads).

Fig. 7 illustrates a typical differential scatter plot for white blood cells after treatment with a saponin/formic acid lysis solution. The ellipses in the figure illustrate the two-dimensional Gaussian probability profiles and the relative lymphocyte, monocyte and granulocyte populations were obtained from these probability distributions. The data in this differential scatter plot were analyzed as for Fig. 8.

Fig. 8 illustrates the correlation of the relative WBC distributions as a function of the measurements obtained from a reference full blood analyzer. The covariance factors between the results obtained from the impedance cytometer according to embodiments of the present invention and the reference device are illustrated in the legend. For one sample, the same experiment was repeated five times, each time preparing a new lysate and a fully cleaned chip.

Fig. 9 shows how the CD4+ populations are identified due to the labeling with antibody coated beads. The lymphocytes and monocytes are clearly discriminated in the impedance plot.

Fig. 10 and Fig. 11 show an example of data from an experiment. Fig. 10 shows a plot of the lymphocytes and neutrophils before labeling. Fig. 11 shows a plot of the blood cells after labeling with CD4 antibody functionalised beads.

Fig. 12 are FACS scatter plots for lysed whole blood and lysed whole blood incubated with CD4 beads. The ellipse shows the location of the CD4+ T-lymphocyte population with attached latex beads.

Fig. 13 shows FACS data from a sample of lysed whole blood which was both labeled with fluorescent antibodies (against CD4 and CD14) and incubated with CD4 beads.

Fig. 14 is an IS scatter plot of the opacity (|Z| @ 10007 kHz / |Z| @ 503 kHz) versus low frequency magnitude (503 kHz) showing the various populations.

Fig. 15 shows calibration data for an impedance micro-cytometer according to embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

DETAILED DESCRIPTION OF ILLUSTRATIVE EMBODIMENTS

In one aspect, the present invention provides the use of a low-cost simple impedance flow cytometric analysis method in combination with impedance labeling to identify and count cells, e.g. blood cells, expressing a specific marker, for example to identify and count the CD4+ T-lymphocytes in human blood.

The detailed description hereinafter is given for the particular embodiments of identifying and counting CD4+ T-lymphocytes in blood; however, the present invention is not limited thereto but includes in general the identification, discrimination and counting of particles, which may include (non-exhaustive list) bacteria, viruses, non-biological particles, cells, dissociated tissue, nano beads with viruses attached.

Blood in general, and human blood in particular, comprises blood cells which fall into any of the following three categories: red blood cells or erythrocytes for transport of oxygen to body tissues, white blood cells or leukocytes for producing antibodies to defend the body against infections and foreign materials, and platelets or thrombocytes for blood clotting. The three categories of blood cells are suspended in plasma. Plasma is 90% water, constitutes about 55% of the blood volume and contains various proteins (e.g. albumin, fibrinogen, globulins and other clotting proteins).

Five different and diverse types of leukocytes exist: neutrophils (40-75% of the leukocytes), eosinophils (0-7% of the leukocytes), basophils (0-1 % of the leukocytes) (these three being grouped under the name granulocytes), lymphocytes (20-45% of the leukocytes) and monocytes (3-11 % of the leukocytes). The number of leukocytes in the blood is often an indicator of disease. There are normally between $4 \times 10^9$ and $1.1 \times 10^{10}$ white blood cells in a liter of blood.

Lymphocytes comprise natural killer cells (NK cells), T cells and B cells. The function of T cells and B cells is to recognize specific "non-self" antigens (antigens in bacteria), during a process known as antigen presentation. Once the T cells and B cells have identified an invader, they generate specific responses that are tailored to maximally eliminate specific pathogens or pathogen infected cells. B cells respond to pathogens by producing large quantities of antibodies when they neutralize foreign objects like bacteria and viruses. In response to pathogens some T cells, called helper T cells produce cytokines that direct the immune response while other T cells, called cytotoxic T cells, produce toxic granules that induce the death of pathogen infected cells.

The cluster of differentiation, also called cluster of designation and often abbreviated as CD, is a protocol used for the identification and investigation of cell surface molecules present on leukocytes. There are approximately 250 different CD proteins. The CD system is commonly used as cell markers, allowing cells to be defined based on what molecules are present on their surface. These markers are often used to associate cells with certain immune functions.

CD molecules are utilized in cell sorting using various methods including flow cytometry. Cell populations are usually defined using a '+' (positive) or a '-' (negative) symbol to indicate whether a certain cell fraction expresses or lacks a CD molecule. Two commonly used CD molecules are CD4 and CD8, which are, in general, used as markers for helper and cytotoxic T cells, respectively. Human immunodeficiency virus (HIV) binds CD4 and a chemokine receptor on the surface of a T helper cell to gain entry. The number of CD4 and CD8 T cells in blood is often used to monitor the progression of HIV infection.

HIV infection leads to a progressive reduction in the number of T cells possessing CD4 receptors. Therefore, medical professionals refer to the CD4 count to decide when to begin treatment for HIV-infected patients. CD4 tests measure the number of T cells expressing the CD4 receptor on their surface. Normal blood values are more than $1 \times 10^9$/L. Results are usually expressed in the number of cells per microliter of blood. Patients often undergo treatments when the CD4 count reaches a low point, around 200-350 cells per microliter.

Flow cytometry is a technique for studying, e.g. counting, sorting and examining chemical and/or physical properties of microscopic particles, for examples cells, which are suspended in a fluid stream.

Embodiments of the present invention make use of flow cytometry for performing impedance spectroscopy.

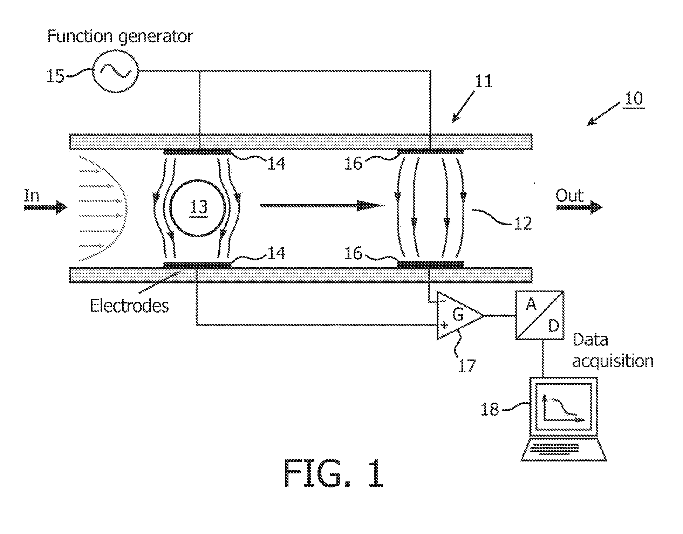

Fig. 1 shows a diagram of the single cell analysis system 10 according to embodiments of the present invention. A micro fluidic device 11 is provided, containing a micro-channel 12 through which cells 13 flow which are suspended in a suspending fluid. This is different from prior art analysis systems where cells are immobilized on a fixed substrate for being counted. A first pair of micro-electrodes 14 are fabricated in the channel 12, and these are connected to a small signal AC voltage generated by a function generator 15. The ratio of this applied voltage to the measured current flowing through the micro-channel 12 between the micro-electrodes 14 is used to determine the electrical impedance of a single cell 13 passing between the micro-electrodes 14.

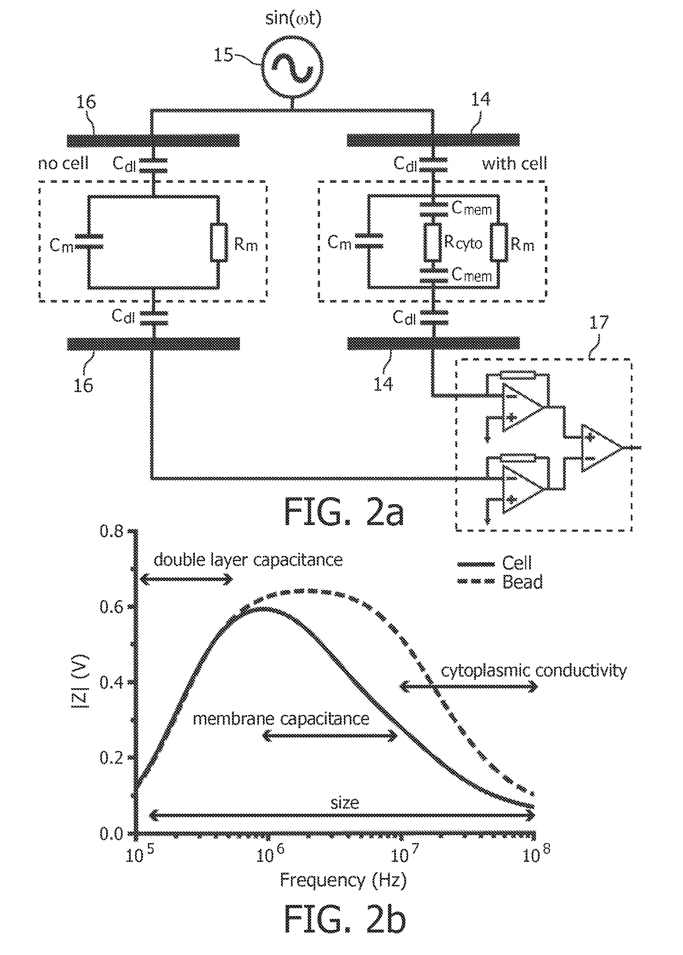

As shown in Fig. 1 and Fig. 2, two pairs of micro-electrodes 14, 16 are used to define two closely positioned detection volumes, both connected to the same function generator 15, hence receiving the same AC voltage, so that a differential measurement is made on individual cells 13. The first pair of electrodes 14 measures the electrical signal from the cell 13 whilst the second pair of electrodes 16 acts as a reference and measures the signal from the micro-channel 12 with suspending fluid but without cell 13 being present. The electrical signals may be measured using custom-made electronics. This double measurement method significantly reduces measurement noise and drift which may arise from variations in temperature or composition of the suspending fluid.

In use, cells 13 in suspension are pumped through the micro fluidic channel 12 of the microfluidic device 11 using a syringe pump (not illustrated) at a pre-determined rate, e.g. a rate of approximately 100 cells 13 per second. As the cells 13 pass through the channel 12 they perturb the electric field in the detection volumes, thus subsequently creating a positive signal (when the cell 13 passes the first pair of electrodes 14) and negative signal (when the cell 13 passes the second pair of electrodes 16); these signals are processed to provide the impedance. The time between the two peaks (positive and negative) in the measured signal corresponds to transit time of the cell 13 between the electrodes 14, 16. In accordance with embodiments of the present invention, high speed impedance analysis is performed at two simultaneous frequencies and data from the system 10 is analyzed using analysis software running on an analysis device 18. The analysis device 18 may comprise a calculation unit for determining the impedance of a blood cell present between the pair of electrodes, the calculation unit being adapted for determining the impedance of the blood cell at the first frequency and at the second frequency, for correlating the impedance at the first frequency to the opacity being the ratio of the high frequency impedance to the low frequency impedance, and for discriminating from this correlation at least the labeled blood cells expressing a specific marker.

The dielectric properties of cells 13 in suspension are described using Maxwell's mixture theory, that characterizes the relationship between the equivalent complex permittivity of a heterogeneous system and the properties of individual particles and suspending medium.

For a suspension of spherical particles (e.g. cells 13) in a medium, the equivalent complex permittivity of the mixture $\varepsilon_{mix}$ is given by

$$\tilde{\varepsilon}_{mix} = \tilde{\varepsilon}_m \frac{1 + 2\Phi f_{CM}}{1 - \Phi f_{CM}}$$

where $f_{CM} = \left( \dfrac{\tilde{\varepsilon}_p - \tilde{\varepsilon}_m}{\tilde{\varepsilon}_p + 2\tilde{\varepsilon}_m} \right)$ and $\tilde{\varepsilon}_m$ and $\tilde{\varepsilon}_p$ are the complex permittivity of the suspending medium and the cell 13

respectively. $\Phi$ is the volume fraction, i.e. the ratio of the volume of the cells 13 to the detection volume. This model is based on the assumption that the cells 13 are solid homogeneous objects. It can be extended to the case of a biological cell using the shell model, which treats the particle as a homogeneous object surrounded by a thin membrane. Now $\tilde{\varepsilon}_p$ is given by:

$$\tilde{\varepsilon}_p = \tilde{\varepsilon}_{mem} \frac{\left( \dfrac{R}{R-d} \right)^3 + 2\left( \dfrac{\tilde{\varepsilon}_i - \tilde{\varepsilon}_{mem}}{\tilde{\varepsilon}_i + 2\tilde{\varepsilon}_{mem}} \right)}{\left( \dfrac{R}{R-d} \right)^3 - \left( \dfrac{\tilde{\varepsilon}_i - \tilde{\varepsilon}_{mem}}{\tilde{\varepsilon}_i + 2\tilde{\varepsilon}_{mem}} \right)}$$

where $\tilde{\varepsilon}_{mem}$ and $\tilde{\varepsilon}_i$ are the complex permittivity of the cell membrane and cell inside (cytoplasm), respectively. R is the radius of the cell and d (d<<R) is the thickness of the membrane.

Mixture theory describes the behavior of a mixture of cells at low volume fractions, $\Phi < 0.2$, for example $\Phi < 0.1$, and is used to model the dielectric properties of a single cell 13 positioned between two measuring electrodes 14 of a first pair of measuring electrodes. If the geometrical parameters of the system are known (electrode size, channel dimensions, etc.) then the values of permittivity and conductivity of the system can be replaced by equivalent electrical components.

A simplified Equivalent Circuit Model (ECM) for a cell 13 in suspension is shown in Fig. 2(a). The impedance of the cell-free medium is represented by the parallel combination of a resistor ($R_m$) and a capacitor ($C_m$), as in Fig. 2 (a), left hand side. With a cell 13 between the electrodes 14, the circuit is modified, as in Fig. 2(a), right hand side. A cell 13 has a thin insulating membrane and behaves as a capacitor ($C_{mem}$). $R_{cyto}$ is the equivalent resistance of the cell cytoplasm. $C_{dl}$ represents the electrical double layer capacitance at the liquid electrode interface.

The component values in the ECM are determined by the dielectric properties of the cell 13 (membrane capacitance and cytoplasmic resistance), the suspending medium conductivity and permittivity, and the volume fraction. The equations that govern the behavior of the system are detailed hereinbelow.

For a cell 13 held between two planar electrodes 14, and for low volume fraction $\Phi$ (for example < 10%) the individual electrical components can be represented as follows:

$$R_m = \frac{1}{\sigma_m(1 - 3\Phi/2)l\kappa} \qquad\qquad C_m = \varepsilon_\infty l\kappa$$

$$C_{mem} = \frac{9\Phi R C_{mem,0}}{4}\kappa l \qquad\qquad R_i = \frac{4\left( \dfrac{1}{2\sigma_m} + \dfrac{1}{\sigma_i} \right)}{9\Phi\kappa l}$$

With

$$\kappa = \frac{K(k)}{K'(k)} \qquad k = \tanh\left(\frac{\pi w}{2h}\right)$$

In this equation, $\kappa$ is the geometrical cell constant for the impedance sensing region, solved using Schwarz-Christoffel mapping method that takes into account the nonuniform electric field. h is the height of the microfluidic channel 12, w the width and l is the length of the electrode 14. K(k) is the complete elliptic integral of the first kind, K'(k) is the complementary integral and k is the modulus of the elliptic function.

In these equations, the volume fraction $\Phi$ is given by:

$$\Phi = \frac{4}{3}\pi R^3 \frac{1}{wlh}$$

Also

$$C_{mem,0} = \varepsilon_{mem} / d \qquad \varepsilon_\infty = \varepsilon_m \frac{2\varepsilon_m + \varepsilon_i - 2\Phi(\varepsilon_m - \varepsilon_i)}{2\varepsilon_m + \varepsilon_i + \Phi(\varepsilon_m - \varepsilon_i)}$$

where $C_{mem}$ is the specific membrane capacitance (capacitance per unit area), d the thickness of the membrane, $\varepsilon_\infty$ is the permittivity at infinite frequency, $\tilde{\varepsilon}_p$ and $\tilde{\varepsilon}_m$ are the complex permittivities of the cell 13 and suspending medium, respectively.

The complex impedance of the system, $\tilde{Z}_{mix}$, becomes:

$$\tilde{Z}_{mix} = \frac{1}{j\omega\tilde{\varepsilon}_{mix}l\kappa}$$

The electric field in the suspending medium also varies with frequency because of the electrical double layer at the electrode-electrolyte interface. In this work the double layer is simply modeled as an ideal capacitor ($C_{dl}$).

The total impedance of the circuit (including the double layer) is:

$$\tilde{Z}_{mix\_DL} = \frac{2}{j\omega C_{DL}} + \frac{R_m(1 + j\omega R_i C_{mem})}{j\omega R_m C_{mem} + (1 + j\omega R_i C_{mem})(1 + j\omega R_m C_m)}$$

Typical values for the dielectric parameters for the cell 13 are:

$\varepsilon_o$ 8.854$\times 10^{-12}$ Fm-1, d = 5nm, $\varepsilon_m$ = 80 $\varepsilon_o$, $\sigma_m$ = 1.6 Sm-1 $\varepsilon_{mem}$ = 11.0 $\varepsilon_o$, $\sigma_{mem}$ = $10^{-8}$ Sm-1, $\varepsilon_i$ = 60 $\varepsilon_o$, $\sigma_i$ = 0.6 Sm-1. In the simulations, the actual values for cell parameters were taken from Table 1. Typical geometric dimension for the micro fluidic device 11 are w = h = l = 20 $\mu$m. Actual values are obtained from measurement of individual microfluidic devices.

From the above, it can be seen that the impedance at low frequencies is dominated by the cell membrane ($C_{mem}$). As the frequency of the applied field increases, this capacitor becomes short circuited and the cell behaves as a resistor ($R_{cyto}$), with a value proportional to the cytoplasm conductivity. The capacitive reactance of the cytoplasm has little effect on the response at high frequencies and is ignored.

The above equations were used to calculate the electrical impedance of a single cell 13 in suspension. A full electrical circuit analysis was performed using PSPICE. The model includes the electrical characteristics of the microfluidic device 11, the electrical double layer, the cell 13 in the suspending medium and the components of the amplifier circuit 17. Fig. 2(b) shows how the magnitude of the impedance of an arbitrary cell 13 and a latex bead (of similar size) vary with frequency. At low frequencies the system is dominated by the electrical double layer. In the frequency range 1 MHz to 10 MHz the impedance of the cell 13 decreases rapidly due to the (Maxwell-Wagner) interfacial relaxation of the cell membrane (in dielectric spectroscopy this is termed the β-dispersion). No such relaxation is measured for a bead and the impedance remains substantially constant. For the cell 13, at higher frequencies the properties of the circuit are dominated by the cytoplasmic resistance and also parasitic capacitances in the electrical circuit. Fig. 2(b) shows that the membrane capacitance has most influence on the response in the 1 to 3MHz region.

In order to characterize different leukocyte sub-populations and determine the optimal frequencies for discrimination, the frequency-dependent impedance of purified T-lymphocytes, monocytes and neutrophils were measured.

The eosinophils, basophils neutrophils are grouped together as granulocytes because they are detected as one group. The eosinophils and basophils make up a small percentage and special techniques are required for the separate identification and enumeration. In each case, the properties of a sample of 5.62 μm diameter latex beads were also measured as a reference.

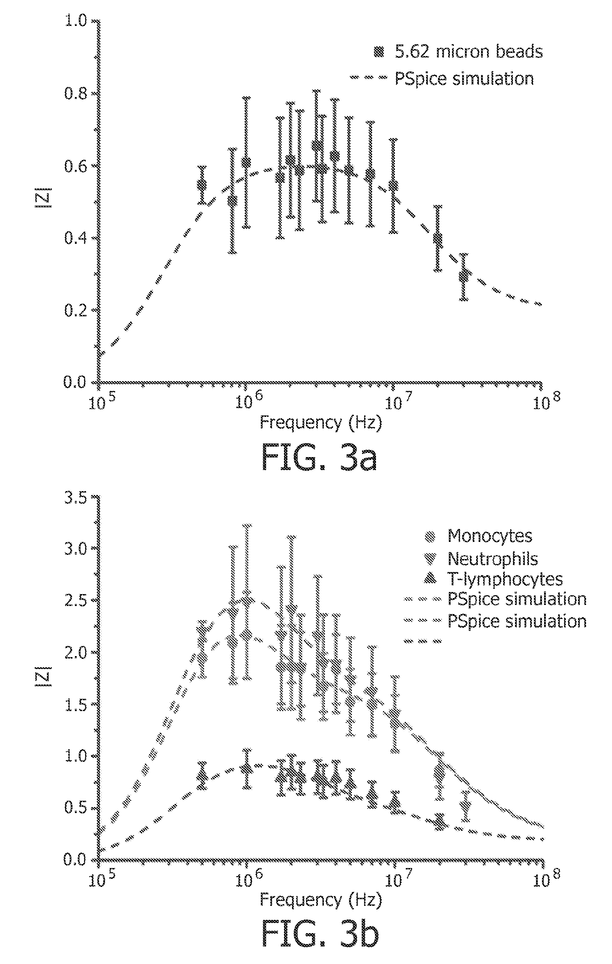

Purified populations of cells 13 and/or latex beads (suspended in physiological saline, PBS (phosphate buffered saline)) were flowed through the micro fluidic device 11 of the cell analysis system 10 and the magnitude of the impedance was recorded at several discrete frequencies. Fig. 3(a) shows the impedance magnitude for 5.62 μm diameter latex spheres as a function of frequency. The data were acquired by simultaneously applying two frequencies to the electrodes 14, 16. A first frequency (the reference) was held at 503 kHz. The low frequency acts essentially as DC. 503kHz is a preferable value, 100kHz to 800kHz is good and DC to 1MHz would work also, whilst the second frequency was changed in discrete steps between 500 kHz AND 50 MHz. Each data point in Fig. 3(a) is the mean value and standard deviation of the impedance signal for approximately 1500 individual events, measured at that particular frequency value for the second frequency. The complete data set shown in Fig. 3(a) is the average frequency-dependent impedance of the 5.62 μm beads. Each set of measurements (1500 beads) takes approximately 2 minutes; there are 14 data sets in the figure, and a complete spectrum takes 30 minutes to record. The transit time for the beads was approximately 1 ms (a particle velocity of 60 mm/sec). When each bead passed through the electrodes 14, 16 a bipolar voltage signal was recorded. This signal was processed to provide the mean peak value; no further signal processing was applied to the data.

Fig. 2(b) shows the data from the PSPICE model, using the equivalent circuit with a double layer capacitance $C_{dl}$ of 60 pF, and including the transfer functions of the various amplifiers (obtained from the manufacturers). The fit (dashed line) was calculated with a bead permittivity of 2.5 $\varepsilon_o$, conductivity of 0.36 mS/m (surface conductivity = 1.2 nS) with PBS (phosphate buffered saline) suspending medium. The data are well described by the equivalent circuit model.

The frequency-dependent properties of the three leukocyte sub-populations were measured in the same way, as illustrated in Fig. 3(b). Also shown are the PSPICE simulations, performed using the equivalent circuit, with values for dielectric parameters taken from Yang J., Huang Y., Wang X-B., Becker FF. and Gascoyne, PRC (2000), "Differential analysis of human leukocytes by dielectrophoretic field-flow-fractionation", Biophys J. 78:2680-2689, as summarized in Table 1.

Table 1

|  | Diameter (μm) | Membrane Capacitance (mF/m$^2$) | Cytoplasm conductivity (mS/m) | Cytoplasm permittivity |
|---|---|---|---|---|
| Lymphocyte | 6.58±0.7 | 10.5 ± 3.1 | 0.65±0.15 | 154.4±39.9 |
| Monocyte | 9.26±0.72 | 15.3 ± 4.3 | 0.56±0.1 | 126.8±35.2 |
| Granulocyte | 9.42±0.46 | 11 ± 3.2 | 0.6±0.13 | 150.9±39.3 |

The data in Fig. 3(b) show that the impedance magnitude varies with cell size, particularly at low frequencies (e.g. the 503 kHz reference frequency). Also variations due to differences in membrane capacitance are evident. T-lymphocytes are much smaller than monocytes or neutrophils, and can be discriminated solely on the basis of size. Table 1 shows that monocytes and granulocytes (including neutrophils, eosinophils and basophils) are of similar size and are difficult to distinguish based on the magnitude of the low frequency impedance. However, their membrane capacitance is different, implying that discrimination should be possible in the mid-frequency band (cf. Fig. 2(b)). The PSPICE fit (dashed lines) confirms this, where the maximum difference in impedance between the monocytes and neutrophils is in the 1 to 3MHz window. The mean values of the experimentally determined impedance also indicate that discrimination between monocytes and neutrophils should be possible in this frequency window. However, there is a large distribution in both size and membrane capacitance for these cells, hence the discrimination is not straight forward.

From Fig. 3, it is clear that the optimum frequency for discrimination based on size is around 500 kHz. In the range 1-10 MHz differences in membrane capacitance dominate. To determine the potential for discrimination, impedance analysis of cells was performed by mixing purified cells in equal ratios (in a buffer solution such as PBS containing 0.5 % BSA (bovine serum albumin) and 2 mM EDTA (ethylenediaminetetraacetic acid)). Measurements were made using two probe frequencies simultaneously, a lower frequency, and a higher frequency, e.g. 503 kHz and 1.707 MHz.

For independent verification, monocytes and neutrophils were labeled with fluorescent labels, e.g. FITC conjugated anti-CD 14 antibody for monocytes and Alexa700 conjugated anti-CD 16 antibody for neutrophils. The fluorescent signal identified each cell and enabled correlation of the measured impedance signal with cell phenotype.

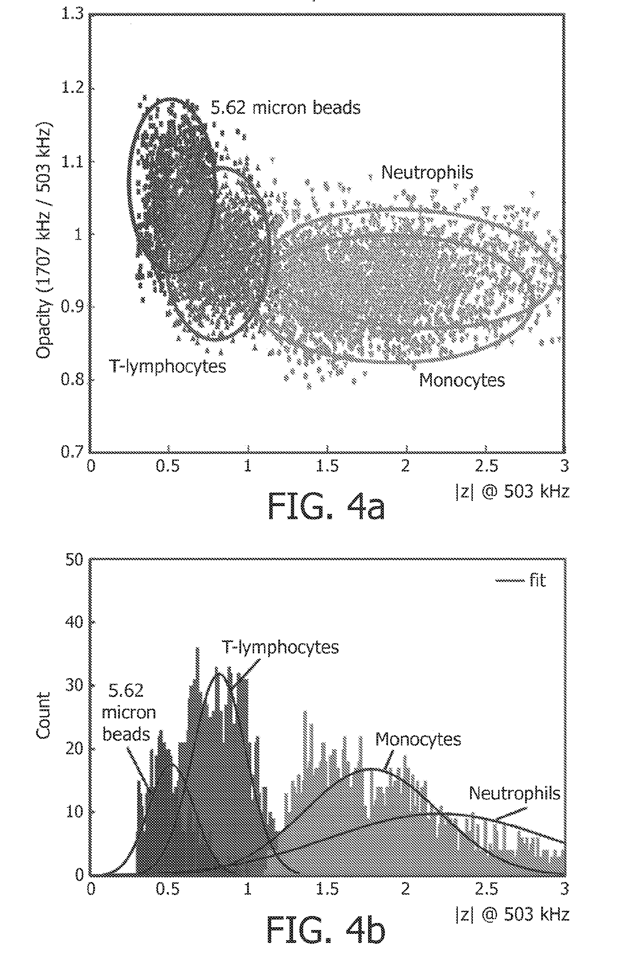

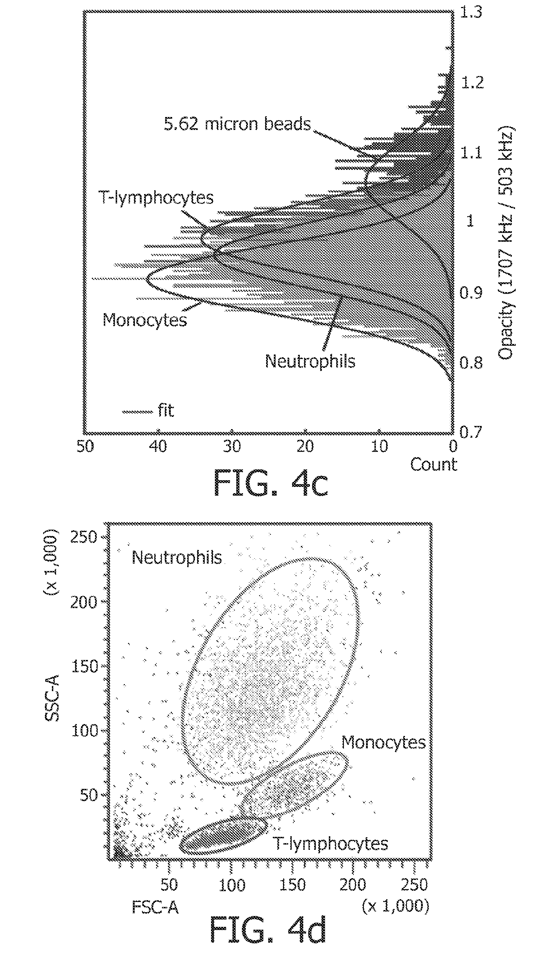

Fig. 4 shows data from a mixture of the three different cell types (T-lymphocytes, monocytes and neutrophils) and 5.62 $\mu$m diameter beads. In this plot the impedance is displayed as a scatter plot of opacity ($|Z|$@ 1707kHz/ $|Z|$@503kHz) vs low frequency impedance ($|Z|$@503kHz). Opacity is the ratio of the high frequency to the low frequency impedance, and provides a parameter which is independent of cell size, thus reflecting changes in the cell membrane. The monocytes and neutrophils populations are color-coded according to the fluorescence. In the graph, different cell populations are encircled by a "best-fit ellipse". The scatter plot (Fig. 4(a)) shows that T-lymphocytes can be discriminated from a group comprising monocytes and neutrophils on the basis of size (low frequency impedance). Also shown are the low frequency impedance magnitude (Fig. 4(b)) and opacity histograms (Fig. 4(c)), together with data from the same sample as measured on a commercial flow cytometer (FACSAria, Becton Dickinson) (Fig. 4(d)) plotted as side scatter (SSC), a measure of granularity, against forward scatter (FSC), roughly proportional to cell size. A good correlation is found between the low frequency impedance data (size) and FSC (Table 2).

Table 2

|  |  | T-lymphocyte (%) | Monocyte (%) | Neutrophil (%) |
|---|---|---|---|---|
| MACS purified, remixed | FACSAria | 37.8 | 32.3 | 29.9 |
|  | Micro-cytometer | 33.6 | 36.7 | 29.6 |
| Saponin treated whole blood | FACSAria | 24.0 (B & T-lymphocytes) | 6.4 | 71.2 |
|  | micro-cytometer | 24.0 | 4.8 | 69.6 |
| Typical ratios in whole blood |  | 20-45 (total lymphocytes) | 3-11 | 40-75 |

Analysis of the impedance scatter plot (Fig. 4(a)) and histograms (Fig. 4(b) and Fig. 4(c)) shows significant overlap between monocytes and neutrophils in the purified and re-mixed cell populations, suggesting that unambiguous discrimination would not be possible without further modification of the cells.

In whole blood, contrary to the purified populations as indicated above, chemical modification of the white blood cells can be used to enhance the electrical signal and aid discrimination of leukocyte sub-populations. For example, exposure of the whole blood to a lysing agent which effectively alters, on a selective basis, one or more cellular constituents of the complex blood sample to a degree necessary to permit the subsequent isolation, identification and/or analysis of the cellular constituents of interest. An example of such a lysing agent may for example be saponin in an organic acid (formic acid) to which the whole blood may be exposed for a short period of time (< 10 seconds) and which modifies the properties of leukocytes, changing the cell volume and cell membrane characteristics. Other lysing agents may be used for destruction of the red cells. Other lysing agents are described for example in US-5155044 and US-4485175. Conventional techniques for the analysis of leukocytes in whole blood often (but not always) require some form of pre-treatment to lyse erythrocytes. It has been found as an embodiment of the present invention that exposure of whole blood to saponin enables both lysis of erythrocytes as previously reported, and modification of the leukocyte populations to improve their discrimination.

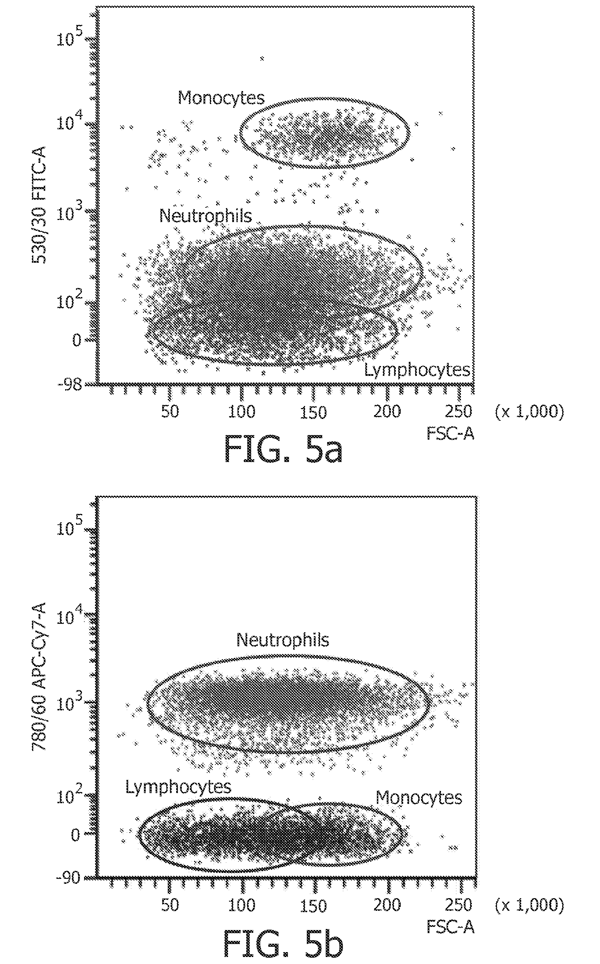

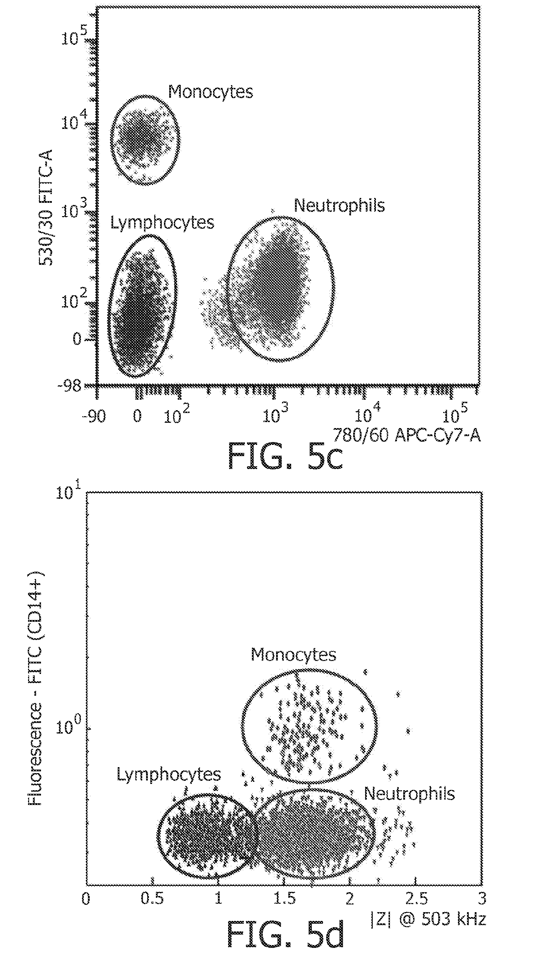

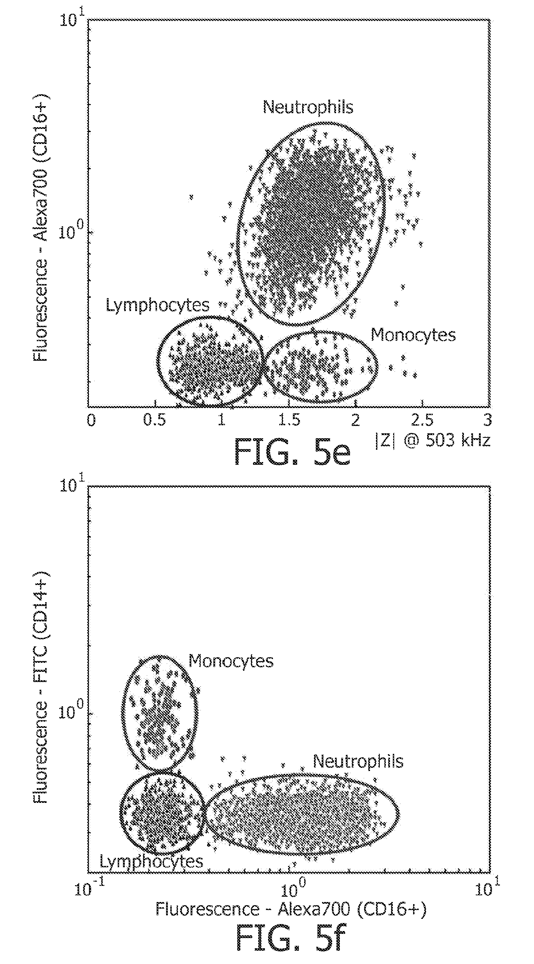

After fluorescently labeling neutrophils (for example with anti-CD16-Alexa700) and monocytes (for example with anti-CD14-FITC), whole blood (50$\mu$l) was exposed to a solution of saponin in formic acid (0.12% v/v formic acid, 0.05% w/v saponin) for 6 seconds, followed by quenching (0.6% w/v sodium carbonate, 3 % w/v sodium chloride) and analysis on the impedance cytometer according to embodiments of the present invention and a flow cytometer (FACS) as known in the art. As shown in Fig. 5(a-c), the saponin/formic acid treatment both removes the erythrocytes and allows the monocytes, neutrophils and lymphocytes to be identified. Fig. 5(d-e) show similar data from the impedance cytometer in accordance with embodiments of the present invention, with each event triggered on the amplitude of the low frequency signal (503 kHz) and false-colored according to the fluorescence signal. The numbers of events for this data set are summarized in Table 2, and correlate with the FACS data.

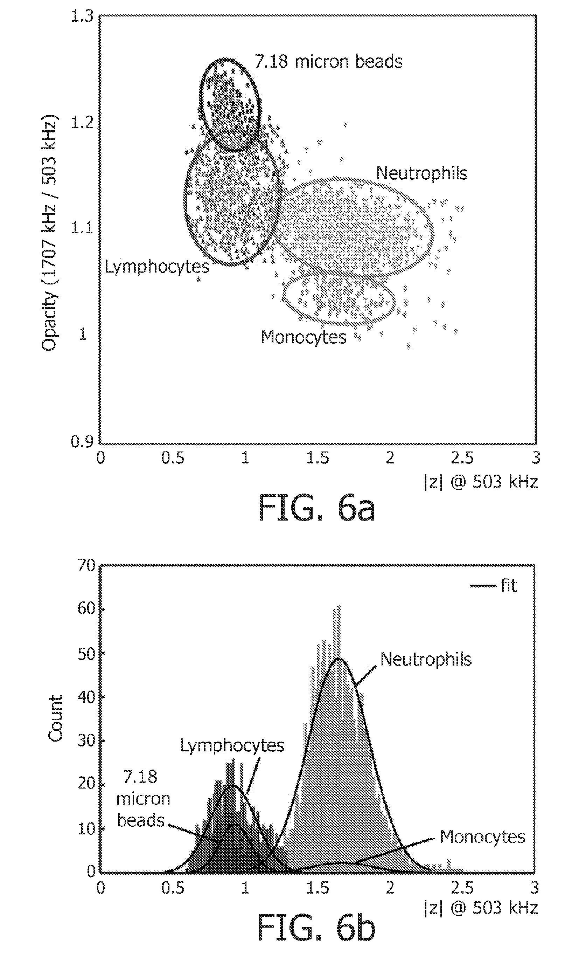

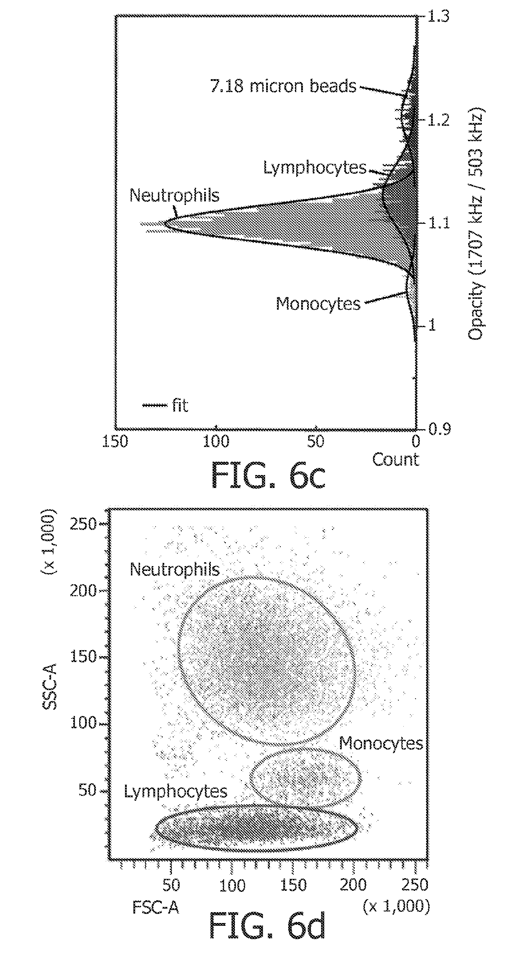

Fig. 6 shows scatter data according to embodiments of the present invention for saponin treated whole blood, (populations, and encircled per type by a "best-fit ellipse" for ease of illustration) with the SSC and FSC data from the prior art FACS in Fig. 6(d). The impedance plot of Fig. 6(a) shows that the three different cell types (lymphocytes, monocytes, neutrophils) can be unambiguously identified solely on the basis of a dual frequency measurement. Cell counts for both the FACS and impedance cytometer were similar and representative of the ratios found in whole blood (table 2). These data were replicated three times using three different patient samples.

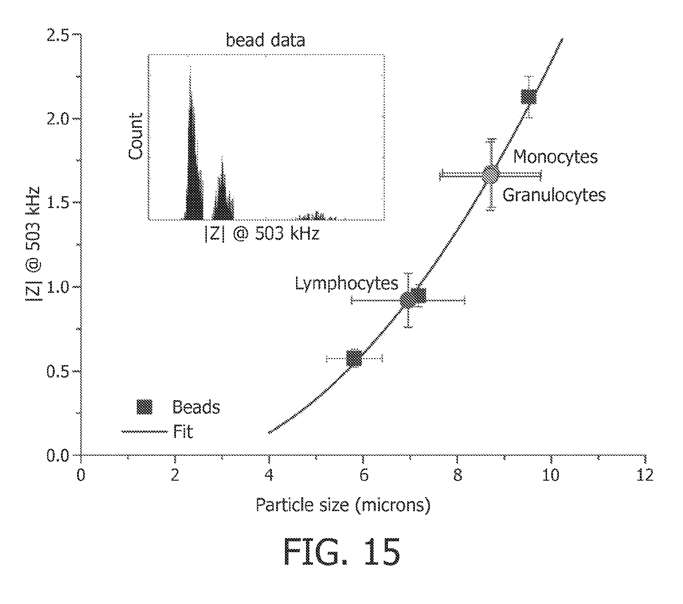

The exact effect of the saponin treatment on the cells is not known, but comparison of Fig. 4(a) with Fig. 6(a) shows that saponin treatment lead to a reduction in the distribution of cell size (impedance at 503kHz). An estimate of the change in cell size can be obtained from the low frequency impedance. The system was first calibrated to allow an unambiguous size analysis of the blood cells. The system was calibrated using three different sizes of latex particles (5.8 $\mu$m, 7.18 $\mu$m and 9.52 $\mu$m). The three sizes of latex beads (5.82 $\mu$m, 7.18 $\mu$m and 9.52 $\mu$m - plotted as black squares in Fig. 15) were mixed and suspended in the saponin/formic acid treated whole blood. The sample was run on the impedance micro-cytometer system according to embodiments of the present invention under the same conditions as the whole blood experiments (data shown in Fig. 5 and Fig. 6). The data are shown in Fig. 15, where the means and standard deviations of the bead populations are plotted as beads size versus low frequency (503 kHz) signal magnitude. A second order polynomial is fitted to the bead data as a calibration curve. The mean signal amplitudes obtained from the whole blood data of Fig. 5 and Fig. 6 is then used with the calibration curve to estimate cell size for the lymphocytes, monocytes and neutrophils. These calibration data were used to size the leukocytes and the results are summarized in Table 1.It can be seen that there is very little change in the mean size of the cells but the variance in the monocyte and neutrophil population distribution is reduced.

Comparison of Fig. 4(a) and Fig. 6(a) also shows that the monocyte and neutrophil populations move relative to each other as a result of the saponin treatment, thus enabling clear discrimination and identification of these two populations. After saponin treatment, the opacity of the monocyte population is reduced relative to the other cells by approximately 5%. The equivalent change in membrane capacitance of the monocytes was calculated (using PSPICE), and corresponds to an increase from approximately 15 mF/m$^2$ to 19 mF/m$^2$. In supplementary experiments, the membrane capacitance of monocytes (magnetically purified, Miltenyi-Biotec MACS) was measured using electrorotation analysis and found to increase from 16 $\pm$ 2 mF/m$^2$ (12 untreated cells) to 19 $\pm$ 3 mF/m$^2$ (saponin/formic acid treated cells). This difference mirrors the change measured using the impedance cytometer according to embodiments of the present invention for monocytes after saponin treatment of whole blood. The mean radius of the cells did not change significantly after saponin/formic acid exposure in either case; the predominant change is the opacity, due to changes in cell membrane structure.

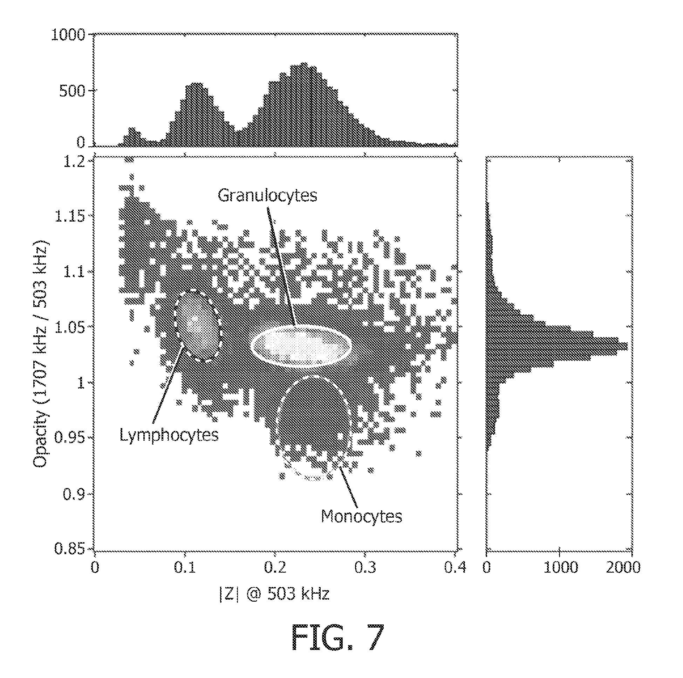

In order to further evaluate the micro fluidic device according to embodiments of the present invention for a differential blood counting, samples were taken from volunteers and measured on a commercial blood analysis instrument and also with the micro-impedance analyzer according to embodiments of the present invention. Over a period of 10 days, 7 separate venous blood samples were obtained from different volunteer subjects (n = 7). Two vials of blood were taken, one was used for experiments and a second vial was sent to a hospital (Mount Alvernia, Guildford UK) for independent full blood count analysis on a Horiba-ABX Pentra DX 120 machine. Fig. 7 shows a scatter plot for one of the samples. Three distributions were identified and fitted to two-dimensional Gaussian probability profiles using a maximum likelihood approach. The ellipses in the figure illustrate these distributions. The relative lymphocyte, monocyte and granulocyte populations were obtained from these probability distributions.

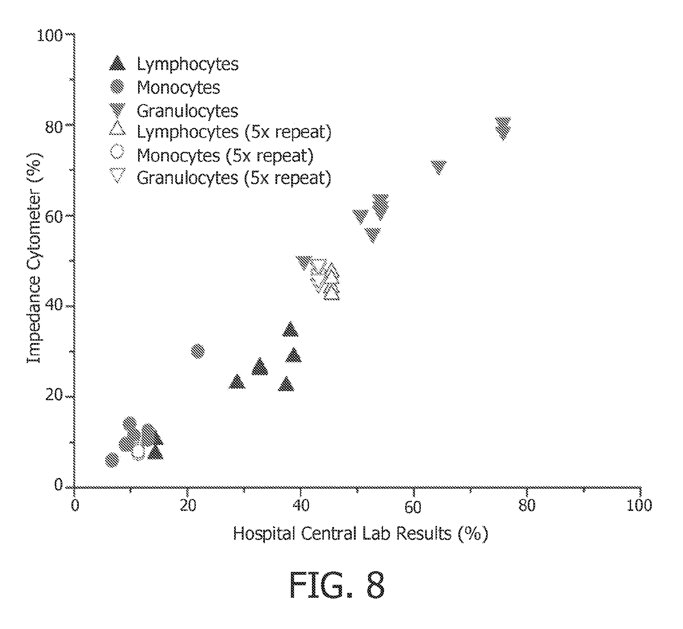

Fig. 8 shows the correlation of the relative WBC distributions with data from the Horiba-ABX Pentra. The monocytes are the least abundant cell species, with fractions between 5%-20%. The granulocytes (predominantly neutrophils) have fractions between 40%-80%. The range of relative fractions covers a significant part, but not all of clinical range. For instance, the normal clinical range of granulocytes is between 20%-90%. The covariance (CV) factors between the results obtained from the impedance cytometer and the reference device are illustrated in the legend. To provide a measure of experimental variance, the experiment with one of the samples was repeated five times, each time preparing a new lysate and using a cleaned chip. This result is indicated by crosses in Fig. 8. From the figure the correlation between the impedance cytometer according to embodiments of the present invention and a commercial full blood analyzer is in the region of 95%.

An analysis method according to embodiment of the present invention for identifying and counting blood cells expressing a specific marker, e.g. for identifying and counting CD4+ T-lymphocytes in human blood, may comprise:

obtaining a sample of blood,
labeling these blood cells in the sample of blood which express the specific marker, e.g. labeling the CD4+ T-lymphocytes in the sample of blood, with an impedance label, for example a dielectric label,

measuring of the labeled sample of blood using a plural, e.g. dual, frequency system to discriminate at least the labeled blood cells expressing the specific marker, e.g. to discriminate at least the labeled CD4+ T-lymphocytes.

The step of measuring the labeled sample to discriminate at least the labeled blood cells expressing the specific marker, e.g. the labeled CD4+ T-lymphocytes, in accordance with embodiments of the present invention may comprise splitting the labeled sample into droplets comprising a single cell,
determining the impedance of the single cell at a first frequency, and
determining the impedance of the single cell at a second frequency, the first frequency being lower than the second frequency, correlating the impedance at the first, lower, frequency to the opacity being the ratio of the second, higher, frequency impedance to the first, lower, frequency impedance, from this correlation discriminating at least the labeled cells expressing the specific marker, e.g. discriminating at least the CD4+ T-lymphocytes.

Labeling the cells expressing the specific marker, e.g. labeling the CD4+ T-lymphocytes, may comprise labeling them with antibody coated beads, e.g. CD4+ antibody coated beads e.g. the commercially available kit Manual CD4 Count beads available from Beckman Coulter, Coulter CD4 Cyto-spheres Reagent. Labeling a particle, e.g. a cell, aids in discrimination due to a change in size of the particle. The inclusion of the beads into the system leads to a small increase in noise. The amplitude of the noise is similar to that seen when platelets are present. This noise signal is easily gated out, as for the 3 part differential measurements.

A method and a system according to embodiments of the present invention require only a tiny sample of blood from a fingerprick and minimal sample preparation that could optionally be automated. This may involve incubation of the whole blood for, for example, 2 minutes with antibody coated beads, e.g. CD4+ antibody coated beads (obtained from a manufacturer such as for example Beckman Coulter. In the particular example given, the CD4+ antibody labels both a subset of the T-lymphocytes and also a subset of the monocytes. An erythrocyte lysis step, for example an automated erythrocyte lysis step using detergents, e.g. using Saponin/Formic Acid or 2% acetic acid in distilled water, may be performed. The Saponin/Formic Acid lysis agent has the advantage that is does not result in significant noise as it does not give rise to presence of RBC debris.

The thus-treated sample may be introduced directly into a micro-fluidic device 11 of a cell analysis system 10, without any other preparatory steps.

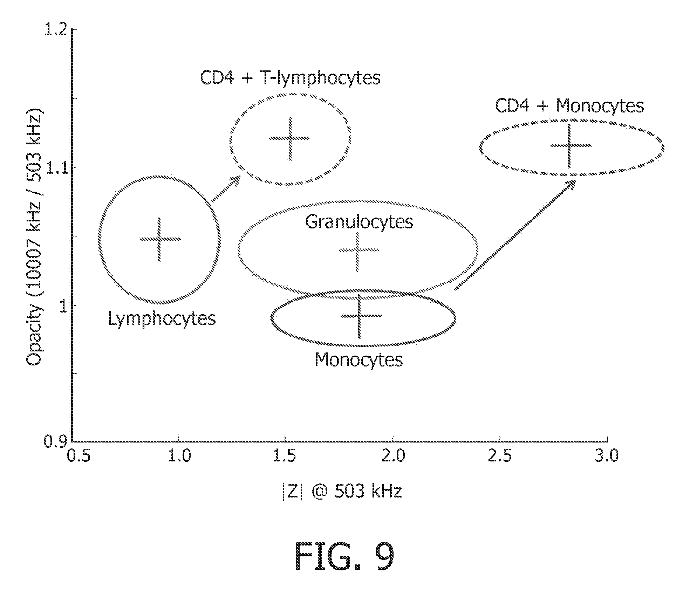

As stated above, the CD4+ antibody labels both a subset of the T-lymphocytes and also the monocytes. The diagnosis of HIV, however, requires accurate counting of only the CD4+ T-lymphocyte subset. This is easy to achieve in accordance with embodiments of the current invention because the CD4+ labeled T-lymphocytes and CD4+ labeled monocytes are very well separated from all other cells and from each other in an opacity versus low frequency impedance scatter plot. The principle of this method is shown in Fig. 9. Fig. 9 shows how the CD4+ populations are identified due to the labeling with antibody coated beads. The lymphocytes and monocytes are clearly discriminated in an impedance plot.

It is an advantage of embodiments of the present invention that, by applying the dual frequency impedance spectroscopy method, on a blood sample in which blood cells expressing a specific antigenic marker is labeled with dielectric labels, CD4+ T-lymphocytes and CD4+ monocytes can be simultaneously discriminated and counted.

According to embodiments of the present invention, measurements of the sample are performed using a dual frequency system to count the monocytes, lymphocytes and neutrophils. Accurate diagnosis is performed by counting the CD4+ cells which can be easily identified as a distinct population because they are dielectrically labeled. In embodiments of the present invention, the absolute CD4+ count may be referenced against the total lymphocyte count. In alternative, less accurate embodiments, the absolute CD4+ count may be referenced against the total leukocyte count which is mostly the sum of the lymphocytes and neutrophils.

A device according to embodiments of the present invention is a microfluidic impedance cytometer that measures the dielectric properties of single cells.

Dual frequency measurements according to embodiments of the present invention enable discrimination of the cells according to both membrane capacitance and size. Chemical pre-treatment of whole blood using saponin/formic acid may be used to eliminate the erythrocyte population from the sample providing a clean leukocyte sample that is free from contamination with cell debris (including erythrocyte ghosts). The pre-treatment also alters the electrical properties of the monocytes and neutrophils, improving discrimination. The lymphocyte, monocyte and neutrophil populations may all be unambiguously indentified using dual frequency impedance data. Volunteer blood was measured on the micro-cytometer and a hospital haematology analyser, showing excellent correlation.

The robust nature and simplicity of microfluidic impedance cytometry according to embodiments of the present invention offers a range of potential applications in cell analysis, including affordable point-of-care (PoC) diagnostic systems using fluorescent label-free techniques. The savings afforded through the simplicity of the system and the reduction in reagents costs (e.g. no requirement for fluorescent probes, etc.), make it applicable for use in low resource settings such as the developing world. Furthermore, as the dielectric properties of cells can be sensitive to stimuli arising from exposure to drug molecules and a variety of mitogens derived from bacterial and viral products, the technology may find applications in cell cycle analysis and toxicity/viability assays.

The system may also be further refined through the development of dielectric tags to enable cells with similar impedance properties to be distinguished (e.g. CD4+ counts for HIV diagnostics). It is possible to do the equivalent of multi-color fluorescence. I.e. a multiplexed assay with multiple CD makers, each detected using populations of beads with different dielectric signature (because they are different size of different materials).

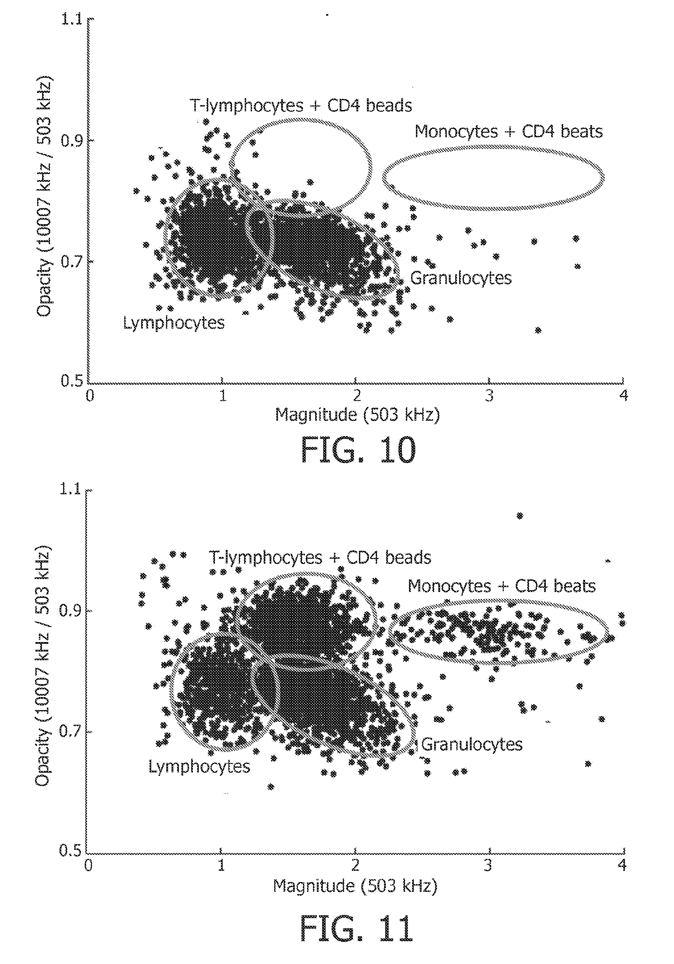

Fig. 10 and Fig. 11 show examples of data from an experiment. Human blood from a healthy donor was treated as outlined above and measured using impedance cytometry in accordance with embodiments of the present invention. Fig. 10 shows the results of a dual frequency impedance measurement of the lymphocytes and granulocytes before labeling of the blood sample. Fig. 11 shows the results of a dual frequency impedance measurement of the blood after labeling with CD4+ antibody coated beads according to embodiments of the present invention. Both the labeled monocytes and T-lymphocytes are discriminated, enabling an easy count to be made and hence a diagnosis.

The method can be used for diagnosis and management of therapy. The absolute values or ratios (e.g. CD4+/CD8+) of antibodies may be determined. The ratio of CD4+ populations to CD8+ populations may be determined by using different tags for both populations, for example one insulating and the other conductive, or tags with different sizes, which allows the identification of different subpopulations of cells.

For patients with AIDS the CD4+ T-cell count provides additional information such as

- Less than 200 / $\mu$l: pneumocystis pneumonia (PCP).
- Less than 100 / $\mu$l: toxoplasmosis and cryptococcal meningitis.
- Less than 75 / $\mu$l: mycobacterium avium complex (MAC).

Experimental results 1:

50 ul of fresh human blood was labeled with fluorescent antibodies against CD4 and CD 14. This allows identification of the cell sub-populations expressing these surface antigens. CD 14 is present on monocytes and CD4 on the surface of a sub-population of T-cells (inducer T-cells) and also at low levels on monocytes.

Cells were also labeled with CD4 coated beads. Both the fluorescent antibody labeling and bead conjugation was carried out prior to an erythrocyte lysis (6 seconds in saponin/formic acid followed by quench). The same samples were then analyzed both by FACS (Fluorescence-activated cell sorter) as known in the art and IS (impedance spectroscopy) in accordance with embodiments of the present invention.

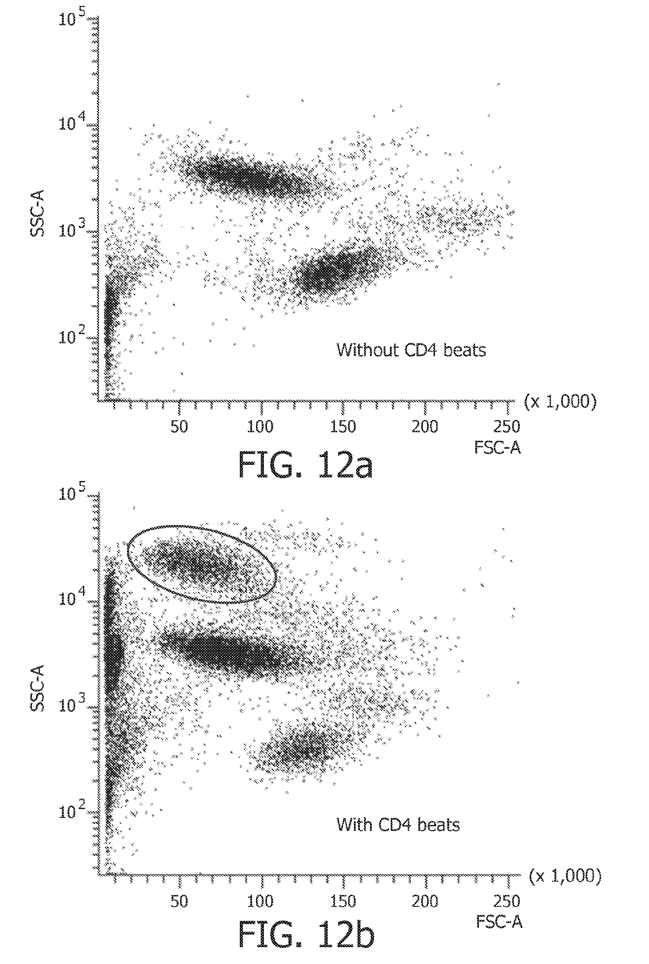

Fig. 12 shows FACS data from a sample of lysed whole blood. Fig. 12(a) is a scatter plot of blood that has not been labeled with CD4 beads and Fig. 12(b) shows a sample of the same blood that was incubated with the CD4 beads. (SSC is plotted on a log scale to allow easy visualization of all the populations).

As shown by these figures, the CD4+ T-cells form a distinct population (outlined in Fig. 12(b)) when complexed with the CD4 beads (-2.2 um diameter), and the SSC signal is increased above the signal from the granulocytes.

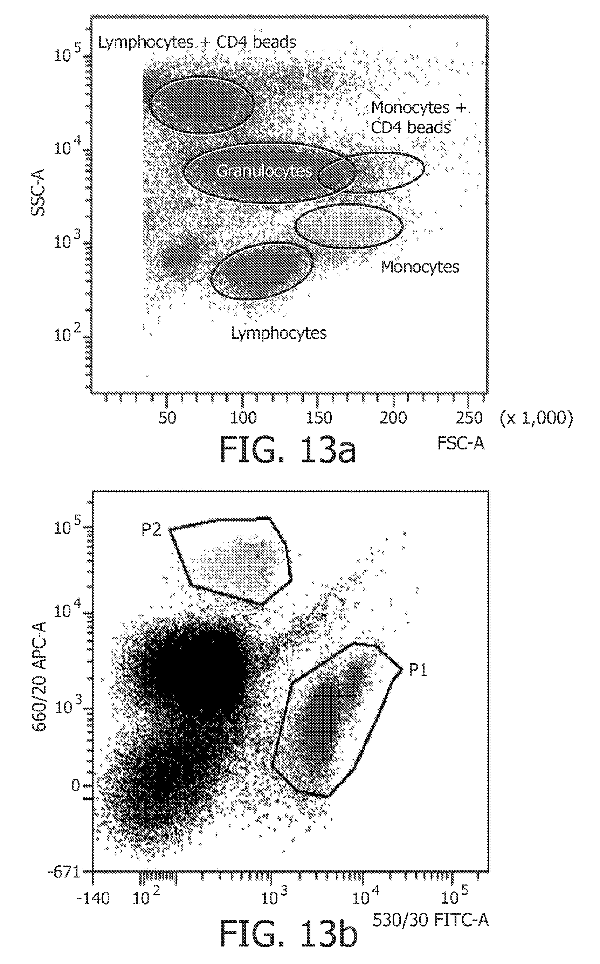

Fig. 13 shows FACS data from a sample of lysed whole blood which was both labeled with fluorescent antibodies and incubated with CD4 beads. The CD4+ cells and the CD 14+ cells can be clearly identified based from their fluorescent signals (regions P1 and P2 respectively in Fig. 13(b)). Gating on these fluorescent populations and plotting FSC versus SSC allows identification of the cells with CD4 beads attached - Fig. 13(a).

The number of cells that were complexed with beads can be enumerated from the FACS data based on the fluorescence and scatter properties of the populations. The CD4+ T-cell population with attached beads is distinct from the bead free lymphocyte population (CD4- T-cells, B-cells, NK, etc). The low level of CD4 expression on the monocytes means that only a small percentage of monocytes bind the CD4 beads (see Beckman Coulter Manual CD4 Count Kit data sheet). The monocytes form two populations; (i) cells with no beads that express CD4+ at very low levels, indicated as "monocytes (no beads)", and (ii) cells complexed with beads that lead to a higher SSC, but clearly not at the same level as the T-cell. This is probably because fewer beads attach to the monocytes (representative of the level of antigen expression) compared to the T-cells - see Fig. 13(a).

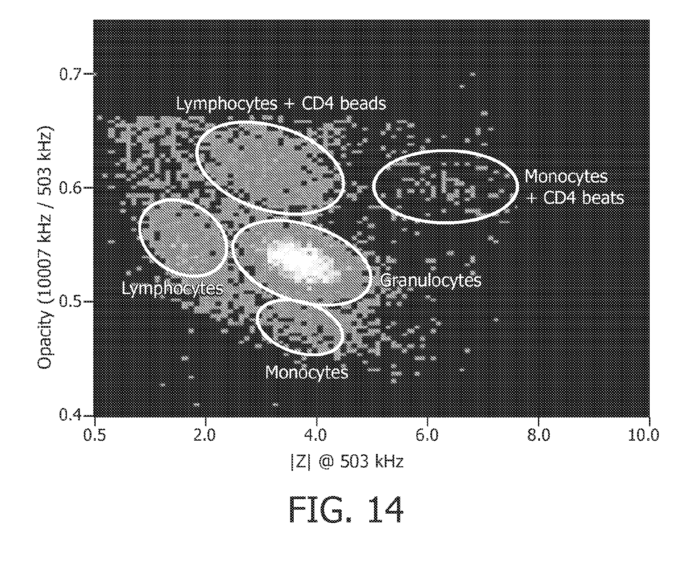

Fig. 14 show IS data plotted as opacity (10MHz/0.5MHz) versus magnitude (0.5MHz) in accordance with embodiments of the present invention. The different populations are visible on the scatter plot. The cells complexed with CD4 beads have an increase in both opacity and size and form a distinct population (circled).

The FACS shows a small change in FSC when the cells are complexed with beads (Fig. 13(a)).

Table 3 shows this data for the different sub-populations, comparing FACS and IS. Although the total counts in both FACS and IS are different, it is possible to compare ratios of cells. The total gated cell counts are shown in brackets. This is a representative data set for one sample of blood. Similar data was obtained on the FACS on three further occasions (different blood samples).

Table 3

| Cell type | FACS (% of total) | IS (% of total) |
|---|---|---|
| T-cells with CD4 beads | **18.0** (11243/62312) | **15.6** (1215/7789) |
| Lymphocytes (no beads) | **12.7** (7914/62312)) | **12.9** (1005/7789) |
| Monocytes with CD4 beads | **0.6** (328/62312) | **0.8** (66/7789) |
| Monocytes (no beads) | **4.3** (2695/62312) | **5.4** (2695/7789) |
| Granulocytes | **64.4** (40132/62312) | **65.4** (5095/7789) |

The CD4+ T-cell count is the critical parameter for diagnosing HIV; comparison of FACS and IS shows that the percentage count is similar. The small error between the two may be due to many reasons, including gating parameters, smaller sample size, or loss of bead-complexed cells in the IS system due to sedimentation. This experiment shows that both technologies produce comparable results using this methodology.

The aim of the above experiment is to the show that the number of CD4+ T-lymphocytes and CD4+ monocytes measured using impedance spectroscopy according to embodiments of the present invention is similar to the number of CD4+ cells measured by commercial FACS.

Experimental results 2:

*Cell preparation*

Blood samples were obtained from healthy donors with informed consent. Blood (5ml) was collected into VacutainerTM tubes (EDTA K3E 15%, 0.12ml, Becton Dickinson). Following collection the blood tubes were placed on a roller and continually mixed at room temperature; all subsequent processing and experimental work was carried out within 12 hours.

*Purified leukocyte sub-populations*

Blood was processed using density gradient separation and magnetically activated cell sorting (MACS, Miltenyi Biotech, Bisley, UK). Anticoagulated whole blood (5 ml) was carefully layered over 5 ml of Polymorphoprep™ (Nycomed, Zurich, Switzerland) and centrifuged for 30 min at 450 x g at room temperature. Following centrifugation the mononuclear cell fraction (mainly monocytes and lymphocytes) and the polymorphonuclear fraction (granulocytes) appear as separate bands on the gradient and were collected into separate tubes. Both cell fractions were washed three times by alternate re-suspending in fresh PBS (containing 0.5% BSA and 2mM EDTA) and pelleting by centrifugation (10 min at 400 x g at room temperature) to remove residual Polymorphoprep™ and contaminating plasma and platelets.

Purified populations of T-lymphocytes (CD3+ cells), monocytes (CD14+ cells) and neutrophils (CD16+ cells) were prepared using magnetic activated cell sorting (MACS). The mononuclear cell sample was split into two portions, one sample incubated with anti-CD3 MACS beads and the other with anti-CD14 MACS beads. The polymorphonuclear cells were incubated with anti-CD16 MACS beads. All incubation and washing steps were carried out in accordance to the manufacturer's guidelines. The individual magnetically tagged cell samples were then fractionated using an AutoMACS™ separation system (Miltenyi Biotec) and the positive cell fraction collected.

To assess the purity of the populations, the samples were labeled with fluorescently conjugated antibodies: anti-CD3-FITC, anti-CD14-APC (both Miltenyi Biotec, Bisley, UK) and anti-CD16-Alexa700 (BioLegend, San Diego, California). Cells were incubated for 30 minutes, followed by washing as above. In all cases matched isotype controls were included as controls. The cell samples were analysed on a FACSAria (Becton Dickinson).

Prior to analysis on the micro-cytometer according to embodiments of the present invention, cells were suspended in PBS (containing 2 mM EDTA and 0.5 % BSA) to a final density of ~ 1 x 106 cells per ml. The purified cell populations were analyzed separately and also as mixtures of the three cell types. The suspending medium was isotonic with a conductivity of 1.6 Sm-1, pH = 7.4 and osmolality = 290 mOs.

*Whole blood analysis (Erythrocyte lysis using Saponin/Formic Acid):*

Whole blood was collected into anticoagulant as described above. 50 $\mu$l of fresh whole blood was incubated with fluorescently conjugated monoclonal antibodies directed against CD14 (FITC) and CD16 (Alexa700) for 15 min in the dark at 4°C. Erythrocyte lysis was performed by the addition of 600 $\mu$l of lysis solution (0.12% v/v formic acid, 0.05% w/v saponin) to the labeled whole blood. The sample was incubated for 6 seconds at room temperature with continual agitation to enhance mixing of the blood and lysis reagent. This lysis reaction was halted by the addition of 265 $\mu$l of quench solution (0.6% w/v sodium carbonate, 3 % w/v sodium chloride). The samples were then analyzed without further processing using both a FACSAria and the micro fluidic impedance cytometer.

*Concordance*

The sample was diluted 1:13 into the lysis solution (as above) for 6 seconds, before dilution (1:1.4) into the quenching agent. The final dilution ratio (with respect to whole blood) was 1:18. Approximately 25 $\mu$L of the solution was pumped through an impedance cytometer chip according to embodiments of the present invention and data measured at 500 kHz and 1.7 MHz simultaneously.

*Microchip*

Chips were fabricated using photolithography and full wafer thermal bonding. Electrical and fluidic interconnections to the chip were made using a connection block machined from PEEK polymer. Fluidic and electrical connections were made by clamping the chip within the block. Spring loaded electrical connectors were used to make contact with the chip electrodes and miniature O-rings used to seal the fluidic connection between the block and chip. The connector block was mounted on a PCB which had the front end impedance detection circuitry. The entire assembly was mounted on an x-y-z stage on an optical bench above a bespoke microscope, allowing alignment of the chip and accurate positioning of the optical detection region and laser spot within the microfluidic channel.

Chips were primed by flowing PBS containing 2 % BSA and 2mM EDTA through the device for 30 minutes. This coated the channel and tubing walls with protein to reduce cell adhesion. Samples of cell suspension were pipetted into a reservoir at the inlet of the chip and sucked through the device at a constant velocity using a syringe pump. A volume flow rate of 5 $\mu$l per minute was used for all experimental work. Between experiments, all equipment was cleaned in 10% sodium hypochlorite and 4M sodium hydroxide.

*Impedance detection*

Two sinusoidal voltages at fixed frequencies (one at 503 kHz and a second varied 0.5 - 30 MHz @ 2.5Vpp) were applied to the both pairs of detection electrodes (see also Fig. 1). A differential current was measured using custom built electronics, and two lock-in amplifiers (SR844, Stanford Research Instruments, USA), one for each frequency. The output from the lock-in consists of the real component (in phase) and imaginary component (90˚ out of phase) of the electrical impedance at each particular frequency. The outputs, together with the photomultiplier signals were sampled at 120 kHz with a 16-bit A-D card (NI6034E, National Instruments) and the data captured and analyzed with software written in LabVIEWTM (National Instruments, USA). The low frequency in-phase impedance signal was used to trigger acquisition of the data. The data were processed and plotted as scatter plots for subsequent analysis in MATLAB (Mathworks Inc., Natick, USA).

*Optical system*

For comparison reasons, an optical measurement was performed at the same time as performing the electrical measurement. Light from a 488 nm (50 mW solid state) and a 633 nm (20 mW HeNe) laser was combined using a dichroic mirror (Chroma Technologies, Rockingham, VT, USA). The beam was expanded using a pair of lenses and passed into the back of an infinity corrected objective lens (x20, NA = 0.7, Nikon). The spot formed at the focal plane of the objective was positioned across the centre of the channel. Fluorescent light was collected via the same objective lens. Dichroic mirrors and bandpass filters (Chroma Technologies, Rockingham, VT, USA) spectrally filtered the light before spatial filtering with lenses and pinholes (100 $\mu$m, Thorlabs) positioned in front of photomultiplier tubes (H7710-13, Hamamatsu). A power supply (C7169, Hamamatsu) was used to adjust the gain on the PMTs and the signals were conditioned and amplified with Hamamatsu amplifiers (C7319, Hamamatsu). Three emission wavelengths were simultaneously measured, 515 nm, 675 nm and >715 nm.

*Flow cytometry*

Conventional flow cytometric analysis of cells was carried out with a FACSAria (Becton Dickinson) equipped with two lasers: 488 nm solid state (20 mW, Coherent® SapphireTM), and 633 nm HeNe (20 mW, JDS UniphaseTM). FACSFlow sheath fluid (Becton Dickinson) was used and samples flow was at a pressure of 70 psi through a 70 $\mu$m nozzle. The instrument was controlled by a PC running FACSDiVa™ software (Becton Dickinson).

While the invention has been illustrated and described in detail in the drawings and foregoing description, such illustration and description are to be considered illustrative or exemplary and not restrictive. The invention is not limited to the disclosed embodiments.

For example, it is possible to operate the invention in an embodiment wherein cell populations can be detected, such as CD3, CD4, CD8, CD14, CD16, CD19, CD21, CD45 or any other cell surface receptor, activation marker. The cells can be human and/or mammalian. Embodiments of the invention can also be applied to non-mammalian cells, e.g. bacteria and yeast cells.

Other examples would be CD64 on neutrophils to sepsis detection or EpCam for the detection of circulating tumor cells in blood or CD34 for stem cells.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

The foregoing description details certain embodiments of the invention. It will be appreciated, however, that no matter how detailed the foregoing appears in text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

**Claims**

1. An analysis method for identifying and counting particles expressing a specific marker, the method comprising:

    obtaining a sample comprising particles,
    labeling the particles expressing the specific marker with an impedance label, measuring the labeled sample using a plural frequency system to discriminate at least the labeled particles expressing the specific marker.

2. An analysis method according to claim 1, wherein labeling the particles expressing the specific marker is performed within a microfluidic system.

3. An analysis method according to claim 1, wherein the step of measuring the labeled sample to discriminate at least the labeled particles expressing the specific marker comprises
    splitting the labeled sample into droplets comprising a single particle, determining the impedance of the single particle at least at a first frequency and at a second frequency, the first frequency being lower than the second frequency,
    correlating the impedance at the first frequency to the opacity being the ratio of the second frequency impedance to the first frequency impedance,
    from this correlation discriminating at least the labeled particles expressing the specific marker.

4. An analysis method according to claim 1, wherein labeling a sample includes incubation of the sample with beads coated with an antibody to the specific marker.

5. A method according to claim 1, furthermore comprising, before measuring the labeled sample, performing a lysis step.

6. A method according to claim 5, the sample being a blood sample, wherein performing a lysis step is performing an erythrocyte lysis step comprising exposing the blood sample to a saponin in an organic acid.

7. A method according to claim 1, wherein measuring the labeled sample is performed in a sample flow.

8. Use of a method according to claim 1, for identifying and counting CD4+ T-lymphocytes.

9. A device for identifying and counting particles expressing a specific marker, comprising
a micro-channel for comprising a sample flow comprising labeled particles expressing the specific marker,
at least one pair of electrodes,
electronic circuitry for applying at least a first AC signal at a first frequency and a second AC signal at a second frequency to the at least one pair of electrodes, the first frequency being lower than the second frequency,
a calculation unit for determining the impedance of a particle present between the pair of electrodes, the calculation unit being adapted for determining the impedance of the particle at least at the first frequency and at the second frequency, for correlating the impedance at the first frequency to the opacity being the ratio of the second frequency impedance to the first frequency impedance, and for discriminating from this correlation at least the labeled particles expressing the specific marker.

10. A device according to claim 9, furthermore comprising a second pair of electrodes adapted for functioning as reference electrodes.

11. A device according to claim 10, furthermore comprising an amplifier circuit adapted for making a differential measurement on a particle present between one of the pairs of electrodes.

12. A device according to claim 9, wherein the micro-channel is adapted for receiving a flow of particles in suspension and directing the flow of particles in suspension between the at least one pair of electrodes for identifying and counting in the flow of particles in suspension the particles expressing a specific marker.

13. An analysis method according to claim 1, wherein the impedance labeling of the sample is carried out on a micro fluidic device.

**FIG. 1**

FIG. 2a

FIG. 2b

FIG. 3a

FIG. 3b

FIG. 4a

FIG. 4b

FIG. 4c

FIG. 4d

FIG. 5a

FIG. 5b

FIG. 5c

FIG. 5d

FIG. 5e

FIG. 5f

FIG. 6a

FIG. 6b

FIG. 6c

FIG. 6d

FIG. 7

FIG. 8

FIG. 9

FIG. 10

FIG. 11

FIG. 12a

FIG. 12b

FIG. 13a

FIG. 13b

FIG. 14

FIG. 15

Europäisches
Patentamt
European
Patent Office
Office européen
des brevets

**EUROPEAN SEARCH REPORT**

Application Number

EP 09 16 2096

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 472 522 A (COULTER ELECTRONICS [US] COULTER CORP [US]) 4 March 1992 (1992-03-04) | 1,3-9 | INV. G01N15/10 G01N33/50 |
| Y | * page 3, lines 32-41; figures 3,7,19 * * page 12, line 1 - page 13, line 11 * ----- | 2,8, 10-13 | |
| Y | CHENG XUANHONG ET AL: "Cell detection and counting through cell lysate impedance spectroscopy in microfluidic devices" LAB ON A CHIP, ROYAL SOCIETY OF CHEMISTRY, CAMBRIDGE, GB, vol. 7, no. 6, 1 June 2007 (2007-06-01), pages 746-755, XP008091009 ISSN: 1473-0197 [retrieved on 2007-05-11] * the whole document * ----- | 8 | |
| Y | HOLMES D ET AL: "Label-Free Differential Leukocyte Counts Using a Microfabricated, Single-Cell Impedance Spectrometer" SENSORS, 2007 IEEE, IEEE, PI, 28 October 2007 (2007-10-28), pages 1452-1455, XP031221347 ISBN: 978-1-4244-1261-7 * the whole document * ----- | 2,10-13 | TECHNICAL FIELDS SEARCHED (IPC) G01N |
| X | US 2009/051372 A1 (SETHU PALANIAPPAN [US] ET AL) 26 February 2009 (2009-02-26) * paragraphs [0010], [0030] - [0033]; figures 1,2 * ----- | 1,8 | |
| | -/-- | | |

The present search report has been drawn up for all claims

| Place of search | Date of completion of the search | Examiner |
|---|---|---|
| Munich | 16 November 2009 | Klein, Marc-Oliver |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 .....................................................................................
& : member of the same patent family, corresponding
    document

EPO FORM 1503 03.82 (P04C01)

**Europäisches Patentamt**
**European Patent Office**
**Office européen des brevets**

## EUROPEAN SEARCH REPORT

| | DOCUMENTS CONSIDERED TO BE RELEVANT | | | |
|---|---|---|---|---|
| Category | Citation of document with indication, where appropriate, of relevant passages | Relevant to claim | CLASSIFICATION OF THE APPLICATION (IPC) | |
| A | HOFFMAN R A ET AL: "FLOW CYTOMETRIC ELECTRONIC DIRECT CURRENT VOLUME AND RADIOFREQUENCY IMPEDANCE MEASUREMENTS OF SINGLE CELLS AND PARTICLES" CYTOMETRY, ALAN LISS, NEW YORK, US, vol. 1, no. 6, 1 January 1981 (1981-01-01), pages 377-384, XP008028058 ISSN: 0196-4763 * the whole document * ----- | 1-13 | | |
| A | US 4 751 179 A (LEDIS STEPHEN L [US] ET AL) 14 June 1988 (1988-06-14) * the whole document * ----- | 1-13 | | |
| T | HOLMES DAVID ET AL: "Leukocyte analysis and differentiation using high speed microfluidic single cell impedance cytometry" LAB ON A CHIP, vol. 9, no. 20, 2009, pages 2881-2889, XP002555068 ISSN: 1473-0197(print) 1473-0189(ele * the whole document * ----- | 1-13 | TECHNICAL FIELDS SEARCHED (IPC) | |

The present search report has been drawn up for all claims

| Place of search | Date of completion of the search | Examiner |
|---|---|---|
| Munich | 16 November 2009 | Klein, Marc-Oliver |

EPO FORM 1503 03.82 (P04C01)

**ANNEX TO THE EUROPEAN SEARCH REPORT
ON EUROPEAN PATENT APPLICATION NO.**

EP 09 16 2096

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2009

| Patent document cited in search report | | Publication date | Patent family member(s) | | | Publication date |
|---|---|---|---|---|---|---|
| EP 0472522 | A | 04-03-1992 | AT | 153758 | T | 15-06-1997 |
| | | | AU | 655154 | B2 | 08-12-1994 |
| | | | AU | 4821890 | A | 01-08-1990 |
| | | | BR | 8907826 | A | 22-10-1991 |
| | | | CA | 1339840 | C | 28-04-1998 |
| | | | CN | 1043566 | A | 04-07-1990 |
| | | | DE | 68928085 | D1 | 03-07-1997 |
| | | | DE | 68928085 | T2 | 15-01-1998 |
| | | | JP | 2795746 | B2 | 10-09-1998 |
| | | | JP | 4503405 | T | 18-06-1992 |
| | | | WO | 9007259 | A2 | 12-07-1990 |
| US 2009051372 | A1 | 26-02-2009 | NONE | | | |
| US 4751179 | A | 14-06-1988 | AU | 4401985 | A | 31-12-1985 |
| | | | CA | 1248856 | A1 | 17-01-1989 |
| | | | DE | 3587036 | D1 | 11-03-1993 |
| | | | DE | 3587036 | T2 | 02-09-1993 |
| | | | EP | 0185048 | A1 | 25-06-1986 |
| | | | ES | 8609727 | A1 | 16-12-1986 |
| | | | JP | 61502277 | T | 09-10-1986 |
| | | | WO | 8505684 | A1 | 19-12-1985 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5155044 A

- US 4485175 A

**Non-patent literature cited in the description**

- **X. Cheng et al.** Cell detection and counting through cell lysate impedance spectroscopy in microfluidic devices. *Lab Chip,* 2007, vol. 7, 746-755

- **Yang J. ; Huang Y. ; Wang X-B. ; Becker FF. ; Gascoyne, PRC.** Differential analysis of human leukocytes by dielectrophoretic field-flow-fractionation. *Biophys J.,* 2000, vol. 78, 2680-2689